(19) Europäisches Patentamt
European Patent Office
Office européen des brevets

(11) Numéro de publication : **0 446 109 B1**

# (12) FASCICULE DE BREVET EUROPEEN

(45) Date de publication du fascicule du brevet : **03.11.93 Bulletin 93/44**

(51) Int. Cl.5 : **C07C 321/14,** C07C 319/14

(21) Numéro de dépôt : **91400566.5**

(22) Date de dépôt : **01.03.91**

(54) **Procédé pour la fabrication du diméthyldisulfure.**

(30) Priorité : **05.03.90 FR 9002715**

(43) Date de publication de la demande : **11.09.91 Bulletin 91/37**

(45) Mention de la délivrance du brevet : **03.11.93 Bulletin 93/44**

(84) Etats contractants désignés : **AT BE CH DE DK ES FR GB GR IT LI LU NL SE**

(56) Documents cités :
**EP-A- 0 269 517**
**EP-A- 0 337 837**
**FR-A- 2 630 105**

(73) Titulaire : **SOCIETE NATIONALE ELF AQUITAINE (PRODUCTION)**
**Tour Elf, 2, Place de la Coupole, La Défense 6**
**F-92400 Courbevoie (FR)**

(72) Inventeur : **Arretz, Emmanuel**
**2 Rue des Cagnes**
**F-64000 Pau (FR)**

(74) Mandataire : **Leboulenger, Jean et al**
**ATOCHEM Département Propriété Industrielle**
**Cédex 42- La Défense 10**
**F-92091 Paris la Défense (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un nouveau procédé de fabrication du diméthyldisulfure.

Une importante voie d'accès aux disulfures organiques consiste en l'oxydation de mercaptans par le soufre en présence d'un catalyseur.

Lorsqu'on met en présence un mercaptan (RSH) et du soufre avec un catalyseur, on obtient le disulfure correspondant selon le schéma réactionnel suivant :

$$2\ RSH + S \xrightarrow{\text{catalyseur}} R\text{-}SS\text{-}R + H_2S$$

mettant en jeu un atome de soufre S pour deux molécules de mercaptan.

La formation du disulfure R-SS-R s'accompagne généralement de la formation de produits secondaires, à savoir des polysulfures de structure analogue au disulfure mais contenant un nombre plus important d'atomes de soufre combiné ($R\text{-}S_n\text{-}R$ avec $n > 2$).

Les réactions conduisant aux polysulfures peuvent être représentées par les équations suivantes :

$$2\ RSH + x\ S \xrightarrow{\text{catalyseur}} R\text{-}S_n\text{-}R + H_2S \quad (x > 1 \text{ et } n = x + 1)$$

$$R\text{-}SS\text{-}R + y\ S \xrightarrow{\text{catalyseur}} R\text{-}S_n\text{-}R \quad (y > 1 \text{ et } n = y + 2)$$

Il est connu que les polysulfures ($R\text{-}S_n\text{-}R$) peuvent être convertis en disulfures (R-SS-R) par réaction avec le mercaptan RSH. Ces réactions peuvent être représentées de la manière suivante :

$$R\text{-}S_n\text{-}R + 2\ RSH \xrightarrow{\text{catalyseur}} R\text{-}S_{n-1}\text{-}R + R\text{-}SS\text{-}R + H_2S$$

$$R\text{-}S_{n-1}\text{-}R + 2\ RSH \xrightarrow{\text{catalyseur}} R\text{-}S_{n-2}\text{-}R + R\text{-}SS\text{-}R + H_2S$$

etc.........

Dans le cas où les polysulfures sont totalement convertis en disulfures, l'équation générale de cette conversion peut être schématisée de la manière suivante :

$$R\text{-}S_n\text{-}R + 2(n-2)\ RSH \xrightarrow{\text{catalyseur}} (n-1) R\text{-}SS\text{-}R + (n-2)\ H_2S$$

Cette réaction de conversion d'un polysulfure en disulfure a été mise à profit dans le brevet US 3 299 146 pour un procédé de préparation du diméthyldisulfure qui consiste à faire réagir le méthylmercaptan sur le diméthyltrisulfure :

$$CH_3\text{-}S_3\text{-}CH_3 + 2\ CH_3SH \rightarrow 2\ CH_3\text{-}SS\text{-}CH_3 + H_2S$$

Les procédés de fabrication continue de dialkyldisulfures à partir d'alkylmercaptans et de soufre, qui sont décrits dans les brevets EP 202420 et EP 337 839 utilisent un réacteur de synthèse dans lequel sont introduits le mercaptan et le soufre. Les polysulfures formés dans la réaction d'oxydation du mercaptan par le soufre

sont, après avoir été séparés du disulfure par distillation, recyclés dans le réacteur de synthèse pour être convertis en disulfure. Ce recyclage continu a pour conséquence d'augmenter la teneur en polysulfures dans le réacteur et de diminuer le rendement en disulfure, et enfin de nécessiter une colonne de distillation de dimensions et de caractéristiques adaptées à des compositions de produits de réaction riches en polysulfures. Dans le cas de la fabrication du diméthyldisulfure à partir de méthylmercaptan et de soufre, les exemples décrits dans les brevets européens précités montrent effectivement que le réacteur de synthèse produit des quantités assez importantes de diméthylpolysulfures.

Selon la présente invention qui concerne un procédé spécifique de fabrication du diméthyldisulfure à partir de méthylmercaptan et de soufre, il a maintenant été trouvé qu'il est beaucoup plus avantageux d'effectuer la réaction de conversion (ou de rétrogradation) des diméthylpolysulfures en diméthyldisulfure dans un réacteur indépendant, les rendements étant, dans ces conditions, beaucoup plus élevés que dans le cas d'un recyclage au réacteur de synthèse. De plus, il a été trouvé que la présence d'hydrogène sulfuré dans les réactifs introduits dans le réacteur de rétrogradation a un effet défavorable sur le rendement de transformation des diméthylpolysulfures en diméthyldisulfure et que l'élimination préalable de l'hydrogène sulfuré permet d'atteindre des conversions en diméthyldisulfure pratiquement totales.

Le nouveau procédé selon l'invention pour la fabrication du diméthyldisulfure à partir de méthylmercaptan et de soufre est caractérisé en ce qu'il comprend deux zones de réaction et une zone de dégazage intermédiaire.

La première zone de réaction est alimentée en réactifs (méthylmercaptan et soufre) qui, en présence d'un catalyseur (éventuellement introduit simultanément avec les réactifs), réagissent entre eux pour donner du diméthyldisulfure et des diméthylpolysulfures.

La zone de dégazage est située en aval de la première zone de réaction et a pour rôle d'éliminer l'hydrogène sulfuré contenu dans les produits bruts liquides sortant de la première zone de réaction. Bien qu'on préfère éliminer l'hydrogène sulfuré le plus complètement possible, on ne sortirait pas du cadre de la présente invention en n'éliminant qu'une partie (au moins 50 %) de l'hydrogène sulfuré. Cette opération de dégazage peut être effectuée, soit par chauffage des produits, soit par entraînement au moyen d'un gaz inerte éventuellement combiné avec un chauffage, à une pression supérieure à la pression atmosphérique pouvant aller jusqu'à 10 bars, de préférence en-dessous de 6 bars.

La deuxième zone de réaction, alimentée en produits provenant de la zone de dégazage après élimination d'au moins 50 % de l'hydrogène sulfuré, a pour but de convertir les diméthylpolysulfures en diméthyldisulfure par réaction avec le méthylmercaptan en présence de catalyseur.

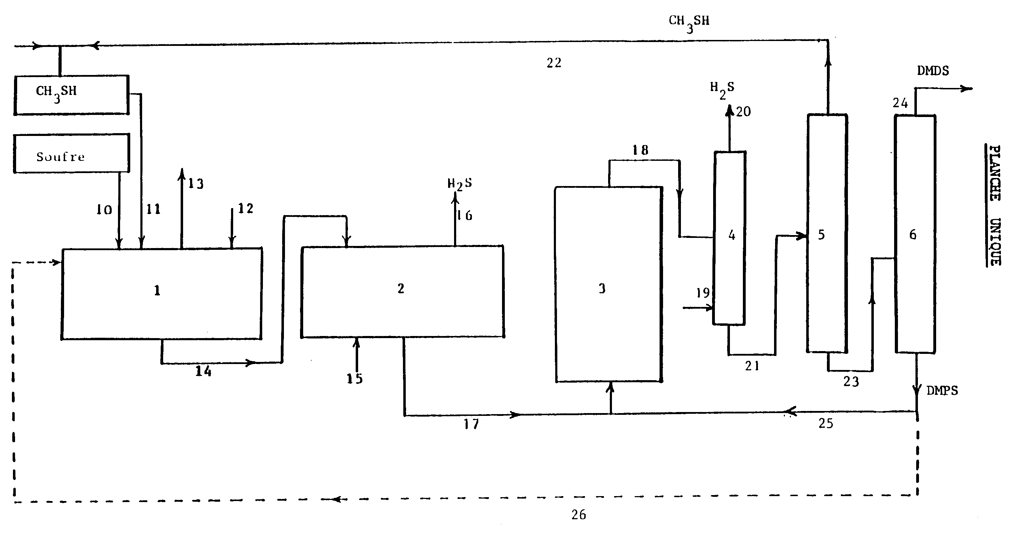

Dans la figure annexée, la représentation schématique d'un mode de réalisation du nouveau procédé de fabrication de diméthyldisulfure comprend un premier réacteur 1 (réacteur primaire), un dégazeur 2, un deuxième réacteur 3 complémentaire (réacteur finisseur), une colonne de dégazage 4 pour l'élimination complète de l'hydrogène sulfuré des produits de réaction avant leur rectification et une section de distillation 5 et 6.

Les réactifs : soufre (liquide ou solide) et le méthylmercaptan (liquide) en excès par rapport à la stoéchiométrie, sont introduits dans le réacteur 1 respectivement par les conduites 10 et 11. Dans le cas où le catalyseur de la réaction est incorporé simultanément, il est introduit par la conduite 12. Les effluents gazeux qui peuvent se former dans le réacteur sont éventuellement éliminés par la conduite 13, et le brut réactionnel liquide, soutiré du réacteur 1, est amené par la canalisation 14 dans le dégazeur 2. Le temps de séjour dans le réacteur 1 est réglé de manière connue en soi de façon à obtenir à sa sortie une conversion quasi-totale du soufre initialement introduit, c'est-à-dire une conversion égale à 100 % ou au moins telle que le soufre non transformé soit solubilisé dans l'effluent liquide.

Le dégazeur 2 est équipé pour éliminer sélectivement par la conduite 16 l'hydrogène sulfuré dissous dans le liquide qui provient du réacteur 1, soit par chauffage, soit par entraînement au moyen d'un gaz inerte introduit par la conduite 15.

Le liquide, débarrassé de l'hydrogène sulfuré, est soutiré en bas du dégazeur et est amené par la canalisation 17 dans le réacteur finisseur 3 dans lequel les diméthylpolysulfures formés dans le réacteur 1 sont convertis en diméthyldisulfure en présence de catalyseur par réaction avec le méthylmercaptan excédentaire. Le temps de séjour dans le réacteur 3 est réglé de manière connue en soi, en fonction de la teneur en diméthylpolysulfures acceptée dans l'effluent, un temps de séjour plus long favorisant la rétrogradation des diméthylpolysulfures en diméthyldisulfure.

Les produits sortant du réacteur 3 sont amenés par la canalisation 18 dans la colonne de dégazage 4 pour l'élimination complète de l'hydrogène sulfuré dissous, soit par chauffage, soit par entraînement au moyen d'un gaz inerte introduit par la conduite 19.

La section distillation est alimentée par l'intermédiaire de la canalisation 21 en produits sortant de la colonne de dégazage 4 pour la séparation dans la colonne 5 du méthylmercaptan contenu dans le produit en vue de son recyclage par la canalisation 22 au réacteur 1. Le produit récupéré en fond de la colonne 5 est

amené par la canalisation 23 dans la colonne 6 en tête de laquelle on récupère par la canalisation 24 le diméthyldisulfure, tandis que les fonds de colonne 6, constitués de diméthylpolysulfures non transformés mélangés à du diméthyldisulfure, sont de préférence recyclés par la canalisation 25 au réacteur finisseur 3. Une variante possible consiste à renvoyer le fond de la colonne 6 dans le réacteur 1 par la canalisation 26 représentée par des tirets sur la figure annexée.

Le dispositif décrit ci-dessus correspond au mode de réalisation le plus simple. L'homme de métier comprendra qu'on ne sortirait pas du cadre de la présente invention en mettant en oeuvre une première zone de réaction constituée de plusieurs réacteurs fonctionnant en parallèle et reliés au même dégazeur ou à une pluralité de dégazeurs constituant une zone de dégazage intermédiaire.

De même, on ne sortirait pas du cadre de la présente invention en mettant en oeuvre une deuxième zone de réaction constituée de plusieurs réacteurs finisseurs. Ainsi, par exemple, pour améliorer le rendement en diméthyldisulfure et éviter le recyclage de diméthylpolysulfures, on peut mettre en oeuvre plusieurs réacteurs finisseurs disposés en série, précédés chacun d'un dégazeur intermédiaire permettant d'éliminer l'hydrogène sulfuré formé dans le réacteur précédent.

Le procédé selon l'invention peut être mis en oeuvre avec différents types de réacteurs, par exemple agités et/ou tubulaires, dont le choix pourra dépendre des conditions de réaction et de la nature des catalyseurs utilisés.

Comme dans les procédés connus, le rapport molaire méthylmercaptan/soufre doit être au moins égal à 2. Un excès important de méthylmercaptan favorisant la sélectivité en diméthyldisulfure, le rapport molaire méthylmercaptan/soufre peut être compris entre 2 et 10, et de préférence est compris entre 3 et 6 pour minimiser les quantités de méthylmercaptan à séparer et à recycler.

Dans chacune des zones de réaction, on opère à des pressions supérieures à la pression atmosphérique. La pression doit au moins être suffisante pour maintenir le méthylmercaptan à l'état liquide et peut aller jusqu'à 50 bars.

Le procédé selon l'invention peut être mis en oeuvre dans un large domaine de température en fonction de la nature des catalyseurs utilisés. La température peut être comprise entre 25°C et 150°C dans le cas de catalyseurs thermiquement stables.

Tous les catalyseurs connus dans l'art antérieur pour l'oxydation de mercaptans par le soufre peuvent être utilisés dans le nouveau procédé selon l'invention, que ce soit des agents basiques organiques ou inorganiques liquides ou solides, tels que bases alcalines, alcoolates alcalins, mercaptides alcalins, combinaisons de bases alcalines avec un mercaptan et un oxyde d'alcène, amines à l'état libre ou fixées à des supports organiques (résines organiques échangeuses d'anions), ou que ce soit des oxydes minéraux de certains métaux comme l'oxyde de magnésium ou des aluminosilicates comme des zéolithes. Les catalyseurs peuvent être identiques dans les deux zones de réactions ou éventuellement différents.

Etant donné les avantages techniques et économiques que représente l'utilisation du soufre à l'état liquide pour son introduction dans les réacteurs de synthèse, le choix du catalyseur peut conditionner le type de réacteur primaire (réacteur 1 dans la figure annexée) à mettre en oeuvre dans le procédé selon l'invention.

Dans le cas où on utilise des catalyseurs de stabilité thermique limitée, comme les résines échangeuses d'anions à fonctions amines tertiaires telles que, par exemple, Amberlyst A21, IRA 93 SP et IRA 94 S, dont la tenue en température ne dépasse pas 100°C, l'introduction de soufre liquide (température de fusion du soufre à partir d'environ 113°C) implique l'emploi d'un réacteur agité, dans lequel le catalyseur est en suspension dans le milieu liquide, comme réacteur primaire (réacteur 1 dans la figure annexée). La réaction entre le méthylmercaptan et le soufre doit être effectuée à une température inférieure à 100°C en présence de ces résines comme catalyseurs.

Dans le cas où on utilise des catalyseurs solides dont la résistance mécanique à l'attrition est faible, l'emploi d'un réacteur agité comme réacteur primaire (réacteur 1 dans la figure annexée) est à éviter. Dans la mesure où ces catalyseurs sont thermiquement stables, leur utilisation en réacteur tubulaire en lit fixe comme réacteur primaire (réacteur 1 dans la figure annexée) est la solution technique la plus appropriée, la température de réaction devant être dans ce cas supérieure à la température de fusion du soufre.

Dans le cas où on utilise des catalyseurs liquides homogènes et stables, le réacteur primaire (réacteur 1 dans la figure annexée) peut être soit de type agité, soit de type tubulaire. Ce type de catalyseur est introduit simultanément avec les réactifs méthylmercaptan et soufre dans le réacteur primaire et dans ce cas il sert de catalyseur au réacteur finisseur (réacteur 3 dans la figure annexée) du procédé selon l'invention, qui peut être soit de type agité, soit de type tubulaire.

Par contre, dans le cas où le réacteur primaire (réacteur 1 dans la figure annexée) contient une charge de catalyseur solide insoluble dans le milieu réactionnel, le catalyseur solide utilisé dans le réacteur finisseur (réacteur 3 dans la figure annexée) peut être identique ou différent. Suivant la nature du catalyseur mis en oeuvre , le réacteur finisseur 3 pourra être de type agité ou tubulaire ; dans le cas d'un catalyseur solide à

faible résistance à l'attrition, le réacteur sera de préférence tubulaire à lit fixe.

Les exemples suivants illustrent l'invention sans la limiter.

**EXEMPLE 1** (comparatif)

**A/ Synthèse continue de diméthyldisulfure à partir de méthylmercaptan et de soufre**

Le réacteur de synthèse, de type agité, est équipé d'une agitation centrale, d'alimentations de méthylmercaptan liquide et de soufre liquide, d'une vanne pneumatique de régulation de pression et d'un système d'évacuation de liquide permettant de maintenir un volume constant dans le réacteur.

Le volume réactionnel est fixé à 300 $cm^3$.

Les essais ont été réalisés avec deux catalyseurs différents : une résine échangeuse d'anions (Amberlyst A21) et une amine (la triéthylamine). Les réactifs sont introduits à ratio molaire méthylmercaptan/soufre égal à 4, le méthylmercaptan avec un débit de 960 g/h et le soufre avec un débit de 160 g/h. La pression dans le réacteur est de 5,5 bars relatifs et la température de réaction est de 40°C.

Pour les essais avec la résine Amberlyst A21, la charge préalablement séchée, utilisée est de 20 g. Dans le cas des essais à la triéthylamine, l'amine est introduite en continu dans le réacteur avec un débit de 1,8 g/h.

Les résultats sont présentés dans le tableau 1. Dans les conditions utilisées, la formation de diméthyldisulfure (DMDS) s'accompagne aussi de la formation de diméthylpolysulfures (DMPS) dont le diméthyltrisulfure est le composé essentiel (> 98 %).

**TABLEAU 1**

| CATALYSEUR | CONVERSION DU SOUFRE(%) | RENDEMENT EN DMDS(%) | PRODUCTION(g/h) | |
|---|---|---|---|---|
| | | | DMDS | DMPS |
| Résine A21 | 100 | 80 | 376 | 63 |
| Triéthylamine | 100 | 78,5 | 370 | 68 |

Dans les deux cas la composition pondérale du mélange DMDS + DMPS en sortie de réacteur est la suivante :

DMDS = 85 %

DMPS = 15 %

**B/ Synthèse continue de diméthyldisulfure à partir de méthylmercaptan et de soufre avec recyclage des diméthylpolysulfures**

Au départ, les conditions opératoires sont les mêmes que celles des essais précédents (paragraphe A), c'est-à-dire :

méthylmercaptan = 960 g/h

soufre = 160 g/h

pression = 5,5 bars relatifs

température = 40°C

Les diméthylpolysulfures (DMPS) formés sont, après avoir été séparés des produits de réaction, introduits dans le réacteur. On observe que le recyclage des polysulfures a pour effet de diminuer le rendement en DMDS et d'augmenter la teneur en DMPS. De manière à obtenir une production stable dans le réacteur, on doit limiter la quantité de polysulfures à recycler. Finalement un régime stationnaire de production s'établit en ne recyclant que la moitié de la quantité des polysulfures récupérés à la sortie du réacteur, quantité qui correspond à la production de polysulfures dans le réacteur.

Ces polysulfures contiennent une proportion relativement élevée de diméthylpolysulfures ayant un nombre

d'atomes de soufre supérieur à 3.

Les résultats de ces essais sont présentés dans le tableau suivant.

**TABLEAU 2**

| CATALYSEUR | RECYCLAGE DMPS (g/h) | SORTIE REACTEUR DMDS + DMPS (g/h) | CONVERSION SOUFRE (%) | RENDEMENT DMDS (%) | PRODUCTION (g/h) | |
|---|---|---|---|---|---|---|
| | | | | | DMDS | DMPS |
| Résine A21 | 162 | 559 | 99,5 | 50 | 235 | 162 |
| Triéthylamine | 160 | 550 | 99,3 | 49 | 230 | 160 |

La composition pondérale du mélange DMDS + DMPS en sortie de réacteur (essai avec résine A21) est de 42 % en DMDS et de 58 % en DMPS, ces polysulfures se répartissant comme suit :

S3 = 45 %
S4 = 10,3 %
S5 = 2,3 %
S6 = 0,4 %

Le recyclage des diméthylpolysulfures formés dans la réaction d'oxydation du méthylmercaptan par le soufre a pour conséquence de diminuer sensiblement le rendement et la productivité en diméthyldisulfure.

## EXEMPLE 2

### Rétrogradation de diméthylpolysulfures en diméthyldisulfure par action du méthylmercaptan

Le réacteur utilisé pour la conversion de diméthylpolysulfures en diméthyldisulfure est un réacteur tubulaire à double enveloppe en acier inoxydable, ayant un diamètre intérieur de 50 mm et contenant une charge de 94 g de résine A21 sèche. Le volume réactionnel est de 300 $cm^3$. Le réacteur est équipé à sa partie inférieure d'alimentations pour l'introduction des réactifs (méthylmercaptan liquide et mélanges de diméthylpolysulfures) et il est relié à sa partie supérieure à une vanne pneumatique de régulation de pression et d'un système d'évacuation en continu du liquide sortant du réacteur.

Les mélanges utilisés comme réactifs de rétrogradation sont des mélanges de diméthyldisulfure et de diméthylpolysulfures qui ont été obtenus à partir des produits récupérés dans les essais de l'exemple 1 effectués dans le réacteur agité en présence de résine A21, après élimination de l'hydrogène sulfuré et du méthylmercaptan que ces produits contenaient.

Les quantités de méthylmercaptan introduites avec ces mélanges correspondent à l'excès de méthylmercaptan non consommé dans la synthèse de ces mélanges DMDS + DMPS dans le réacteur agité qui était alimenté avec un ratio molaire méthylmercaptan/soufre égal à 4.

Les réactifs (méthylmercaptan et mélanges contenant les polysulfures) sont introduits en continu dans le réacteur tubulaire dont la pression est maintenue à 5,5 bars relatifs par de l'azote et la température est réglée à 40°C.

Les produits récupérés à la sortie du réacteur sont analysés et les résultats obtenus sont reportés dans le tableau suivant :

**TABLEAU 3**

| Référence Exemple 1 | REACTIFS | | COMPOSITION PONDERALE DMDS + DMPS | | | |
|---|---|---|---|---|---|---|
| | | | Entrée réacteur | | Sortie réacteur | |
| | DMDS+DMPS (g/h) | Méthylmercaptan (g/h) | DMDS | DMPS | DMDS | DMPS |
| B | 430 | 798 | 42 | 58 | 94 | 6 |
| A | 438 | 528 | 85 | 15 | 98,5 | 1,5 |

## EXEMPLE 3

**Production de diméthyldisulfure par le procédé selon l'invention, au moyen de deux réacteurs (réacteur primaire + réacteur finisseur) et d'un dégazeur intermédiaire**

Les deux réacteurs utilisés pour les essais des exemples 1 et 2 sont associés, le réacteur agité étant le réacteur primaire et le réacteur tubulaire faisant fonction de réacteur finisseur. Entre ces deux réacteurs on adapte un système de dégazage constitué d'un récipient à double enveloppe muni d'un agitateur et surmonté d'une colonne refroidie qui permet de recondenser le méthylmercaptan qui peut être entraîné avec l'hydrogène sulfuré qui doit être dégazé. Suivant les conditions opératoires (température et pression du dégazeur, température de la colonne de condensation) on élimine plus ou moins efficacement l'hydrogène sulfuré dissous dans le produit brut provenant de la sortie du réacteur agité (réacteur primaire), tout en effectuant une condensation pratiquement totale du méthylmercaptan. Cet équipement est complété par une pompe placée entre la sortie du dégazeur et l'entrée du réacteur tubulaire (réacteur finisseur), qui permet d'alimenter ce réacteur en produit liquide traité dans le dégazeur.

Les réactifs (méthylmercaptan liquide avec un débit de 960 g/h et soufre liquide avec un débit de 160 g/h) sont introduits dans le réacteur agité (réacteur primaire), de volume réactionnel de 300 $cm^3$, qui contient 20 g de résine Amberlyst A21 sèche. La pression opératoire est maintenue à 5,5 bars relatifs et la température à 40°C. Le liquide sortant du bas du réacteur est envoyé dans le dégazeur pour être traité. Après traitement dans le dégazeur, le produit est introduit dans le réacteur tubulaire (réacteur finisseur) qui contient une charge de 94 g de résine Amberlyst A21 sèche. La pression dans le réacteur est de 5,5 bars relatifs et la température de 40°C. A la sortie du réacteur, le produit brut est récupéré et analysé.

Des essais ont été effectués avec différents taux d'élimination de l'hydrogène sulfuré dans le dégazeur.

Les résultats de ces essais sont présentés dans le tableau suivant :

**TABLEAU 4**

| REACTEUR PRIMAIRE (sortie) | | DEGAZEUR | REACTEUR FINISSEUR (sortie) | |
|---|---|---|---|---|
| Composition pondérale (%) | | TAUX D'ELIMINATION d'$H_2S$ (%) | Composition pondérale (%) | |
| DMDS | DMPS | | DMDS | DMPS |
| 85 | 15 | 0 | 90 | 10 |
| 85 | 15 | 35 | 93 | 7 |
| 85 | 15 | 70 | 95 | 5 |
| 85 | 15 | 100 | 98,5 | 1,5 |

## Revendications

1. Procédé de fabrication de diméthyldisulfure à partir de méthylmercaptan et de soufre, caractérisé en ce qu'il comprend :
   **(a)** une première zone de réaction dans laquelle le méthylmercaptan et le soufre sont introduits et mis à réagir en présence d'un catalyseur,
   **(b)** une zone de dégazage dans laquelle les produits issus de la première zone de réaction sont traités pour éliminer au moins partiellement l'hydrogène sulfuré dissous, et
   **(c)** une seconde zone de réaction dans laquelle les produits issus de la zone de dégazage sont mis en contact avec un catalyseur.

2. Procédé selon la revendication 1, dans lequel la première zone de réaction est constituée d'un réacteur ou de plusieurs réacteurs disposés en parallèle.

3. Procédé selon la revendication 1 ou 2, dans lequel on introduit, dans la première zone de réaction, le méthylmercaptan sous forme liquide et le soufre sous forme solide ou liquide, de préférence sous forme liquide.

4. Procédé selon l'une des revendications 1 à 3, dans lequel le rapport molaire méthylmercaptan/soufre, étant au moins égal à 2, est compris entre 2 et 10, et de préférence compris entre 3 et 6.

5. Procédé selon l'une des revendications 1 à 4, dans lequel on opère dans la première zone de réaction à une température allant de 25°C à 150°C, sous une pression effective pouvant aller jusqu'à 50 bars.

6. Procédé selon l'une des revendications 1 à 5, dans lequel l'élimination de l'hydrogène sulfuré dissous dans le liquide sortant de la première zone de réaction est effectuée dans la zone de dégazage par chauffage des produits ou par entraînement au moyen d'un gaz inerte, éventuellement combiné avec un chauffage, à une pression supérieure à la pression atmosphérique.

7. Procédé selon l'une des revendications 1 à 6, dans lequel la deuxième zone de réaction peut être constituée d'un ou plusieurs réacteurs.

8. Procédé selon la revendication 7, dans lequel la deuxième zone de réaction comprend plusieurs réacteurs disposés en série, précédés chacun d'un dégazeur intermédiaire.

9. Procédé selon l'une des revendications 1 à 8, dans lequel on opère dans la deuxième zone de réaction

à une température allant de 25°C à 150°C, sous une pression effective pouvant aller jusqu'à 50 bars.

10. Procédé selon l'une des revendications 1 à 9, dans lequel les catalyseurs utilisés dans les deux zones de réactions sont identiques ou différents et sont choisis parmi les agents basiques inorganiques ou organiques liquides ou solides, les oxydes minéraux et les aluminosilicates.

11. Procédé selon l'une des revendications 1 à 10, dans lequel le produit issu de la deuxième zone de réaction est soumis à une élimination complète de l'hydrogène sulfuré dissous, puis à une distillation pour récupérer dans une première colonne le méthylmercaptan non consommé mis en excès dans la première zone de réaction et le recycler dans la première zone de réaction, pour purifier le diméthyldisulfure dans une deuxième colonne et séparer les diméthylpolysulfures non convertis.

12. Procédé selon la revendication 11, dans lequel les diméthylpolysulfures séparés dans la deuxième colonne de distillation sont renvoyés à l'entrée de la deuxième zone de réaction.

**Patentansprüche**

1. Verfahren zur Herstellung von Dimethyldisulfid ausgehend von Methylmercaptan und Schwefel, dadurch gekennzeichnet, daß
   a) in einer ersten Reaktionszone, Methylmercaptan und Schwefel eingesetzt werden und in Gegenwart eines Katalysators zur Reaktion gebracht werden,
   b) in einer Entgasungszone die in der ersten Reaktionszone entstandenen Produkte behandelt werden, um zumindest teilweise den gelösten Schwefelwasserstoff zu entfernen, und
   c) in einer zweiten Reaktionszone, die Produkte, die aus der Entgasungszone kommen, mit einem Katalysator in Kontakt gebracht werden.

2. Verfahren nach Anspruch 1, in dem die erste Reaktionszone aus einem oder aus mehreren parallel angeordneten Reaktoren besteht.

3. Verfahren nach Anspruch 1 oder 2, in dem man in der ersten Reaktionszone Methylmercaptan in flüssiger Form und den Schwefel in fester oder flüssiger Form, vorzugsweise jedoch in flüssiger Form, zuführt.

4. Verfahren nach einem der Ansprüche 1 bis 3, in dem das Molverhaltnis Methylmercaptan/Schwefel, das mindestens 2 beträgt, zwischen 2 und 10, vorzugsweise jedoch zwischen 3 und 6 liegt.

5. Verfahren nach einem der Ansprüche 1 bis 4, in dem man in der ersten Reaktionszone bei einer Temperatur zwischen 25 und 150 °C und bei einem Überdruck bis zu 50 Bar arbeitet.

6. Verfahren nach einem der Ansprüche 1 bis 5, in dem die Entfernung des Schwefelwasserstoffs, der in der Flüssigkeit aus der ersten Reaktionszone gelöst ist, in der Entgasungszone durch Erhitzen der Produkte oder durch Mitreißen mittels eines Inertgases - eventuell kombiniert mit Erhitzen - bei einem Druck oberhalb Atmosphärendruck erfolgt.

7. Verfahren nach einem der Ansprüche 1 bis 6, in dem die zweite Reaktionszone aus einem oder mehreren Reaktoren bestehen kann.

8. Verfahren nach Anspruch 7, in dem die zweite Reaktionszone mehrere in Serie angeordnete Reaktoren umfaßt, und jedem Reaktor ein Entgaser als Zwischenstufe vorgeschaltet ist.

9. Verfahren nach einem der Ansprüche 1 bis 8, in dem man in der zweiten Reaktionszone bei einer Temperatur zwischen 25 und 150 °C und bei einem Überdruck bis zu 50 Bar arbeitet.

10. Verfahren nach einem der Ansprüche 1 bis 9, in dem die verwendeten Katalysatoren in beiden Reaktionszonen gleich oder verschieden sind und aus der Gruppe der flüssigen oder festen, anorganischen oder organischen, basischen Substanzen, der mineralischen Oxide oder der Aluminiumsilikate gewählt werden.

11. Verfahren nach einem der Ansprüche 1 bis 10, in dem das Produkt, das aus der zweiten Reaktionszone hervorgegangen ist, vollständig vom gelösten Schwefelwasserstoff befreit und einer Destillation unterworfen wird, um in einer ersten Kolonne das unverbrauchte Methylmercaptan, das im Überschuß der ersten Reaktionszone zugeführt wurde, aufzufangen, und es in die erste Reaktionzone zurückzuführen, und um das Dimethyldisulfid in einer zweiten Kolonne zu reinigen, und die nicht umgesetzten Dimethylpolysulfide abzutrennen.

12. Verfahren nach Anspruch 11, in dem die Dimethylpolysulfide, die in der zweiten Destillationskolonne abgetrennt wurden, zum Beginn der zweiten Reaktionszone zurückgeführt werden.

## Claims

1. Process for the manufacture of dimethyl disulphide from methanethiol and sulphur, characterised in that it comprises:
   (a) a first reaction region in which methanethiol and sulphur are introduced and reacted in the presence of a catalyst,
   (b) a degassing region in which the products resulting from the first reaction region are treated in order to at least partially remove the dissolved hydrogen sulphide, and
   (c) a second reaction region in which the products resulting from the degassing region are brought into contact with a catalyst.

2. Process according to Claim 1, in which the first reaction region consists of a reactor or of a number of reactors arranged in parallel.

3. Process according to Claim 1 or 2, in which, in the first reaction region, methanethiol is introduced in the liquid form and sulphur is introduced in the solid or liquid form, preferably in the liquid form.

4. Process according to one of Claims 1 to 3, in which the methanethiol/sulphur molar ratio, being at least equal to 2, is between 2 and 10, and preferably between 3 and 6.

5. Process according to one of Claims 1 to 4, in which the reaction is carried out in the first reaction region at a temperature ranging from 25°C to 150°C, under an effective pressure which can range up to 50 bars.

6. Process according to one of Claims 1 to 5, in which removal of the hydrogen sulphide dissolved in the liquid emerging from the first reaction region is carried out in the degassing region by heating the products or by entrainment by means of an inert gas, optionally combined with heating, at a pressure greater than atmospheric pressure.

7. Process according to one of Claims 1 to 6, in which the second reaction region can consist of one or a number of reactors.

8. Process according to Claim 7, in which the second reaction region comprises a number of reactors arranged in series, each preceded by an intermediate degasser.

9. Process according to one of Claims 1 to 8, in which the reaction is carried out in the second reaction region at a temperature ranging from 25°C to 150°C, under an effective pressure which can range up to 50 bars.

10. Process according to one of Claims 1 to 9, in which the catalysts used in the two reaction regions are identical or different and are chosen from inorganic oxides, aluminosilicates and liquid or solid, inorganic or organic, basic agents.

11. Process according to one of Claims 1 to 10, in which the product resulting from the second reaction region is subjected to complete removal of the dissolved hydrogen sulphide and is then distilled in order to recover, in a first column, the unreacted methanethiol introduced in excess into the first reaction region and to recycle it into the first reaction region, and in order to purify the dimethyl disulphide in a second column and to separate the unconverted dimethyl polysulphides.

12. Process according to Claim 11, in which the dimethyl polysulphides separated in the second distillation column are conveyed to the inlet of the second reaction region.

PLANCHE UNIQUE